# EUROPEAN PATENT APPLICATION

(11) **EP 4 421 092 A1**
(43) Date of publication of application: **28.08.2024**
(21) Application number: 22882219.3
(22) Date of filing: 21.03.2022
(51) Int. Cl.: C07K 16/28, C07K 16/46, C12N 5/10, C12N 15/85, C12P 19/34, A61P 35/00, A61K 39/395

(54) **ANTI-CD47-CLDN18.2 BISPECIFIC ANTIBODY AND USE THEREOF**

(30) Priority: 19.10.2021 CN 202111214360
(71) Applicant: Dragonboat Biopharmaceutical (Shanghai) Co., Ltd., Shanghai 201203 (CN); Sanyou Biopharmaceuticals Co., Ltd., Shanghai 201114 (CN)
(72) Inventor: HUANG, Yingfeng, Shanghai 201203 (CN); LANG, Guojun, Shanghai 201114 (CN); TAN, Yongcong, Shanghai 201114 (CN); SHAO, Zhe, Shanghai 201203 (CN); ZHANG, Ruixia, Shanghai 201203 (CN); YAN, Run, Shanghai 201114 (CN); WANG, Tao, Shanghai 201203 (CN); XU, Junyan, Shanghai 201203 (CN); HU, Yuhao, Shanghai 201114 (CN); ZHANG, Wenhai, Shanghai 201114 (CN)
(74) Representative: Reitstötter Kinzebach
(86) International application number: PCT/CN2022/082008
(87) International publication number: WO 2023/065594

(57) **Abstract**

The present invention belongs to the field of biomedicine. Specifically, the present invention relates to an anti-CD47/anti-CLDN18.2 bispecific antibody and the use thereof.

## Description

The present application claims priority to Chinese Patent Application No. 202111214360.8 filed on October 19, 2021 and entitled with "ANTI-CD47-CLDN18.2 BISPECIFIC ANTIBODY AND USE THEREOF", the content of which is incorporated herein by reference in its entirety.

### Technical Field

The present invention belongs to the field of biomedicine. Specifically, the present invention relates to an anti-CD47/anti-CLDN18.2 bispecific antibody and the use thereof.

### Background Art

CD47 is a transmembrane glycoprotein widely expressed on cell surface, belongs to the immunoglobulin superfamily and can interact with signal regulatory protein alpha (SIRPα), thrombospondin-1 (TSP1) and integrins to mediate a series of responses such as cell apoptosis, proliferation and immunization. It has been demonstrated that the phagocytosis of tumor cells can be effectively mediated by blocking the CD47-SIRPα pathway with an anti-CD47 antibody, thereby inhibiting the growth of various hematological malignancies and solid tumors *in vivo.* However, CD47 is highly expressed not only on tumor cells, but also on normal cells, such as red blood cells. Therapies targeting CD47 may cause undesired side effects. Some anti-CD47 antibodies disclosed in the prior art (see, e.g., US 20160304609) bind to red blood cells, which not only causes severe anemia, but also requires an administration dose of up to 30 mg/kg. These characteristics pose a huge challenge to the clinical application of anti-CD47 antibodies.

CLDN18 belongs to the Claudin family, which was discovered by Shoichiro Tsukita et al. in 1998. It is an important molecule for tight junctions of epithelial cells, determines the epithelial permeability, and also plays a role in blocking the diffusion of proteins and lipids on the surface of cell membranes (Gunzel, D. and A. S. Yu (2013) Physical Rev. 932: 525-569). The human CLDN18 gene has two different exons 1 and is subject to alternative splicing after transcription to finally generate two protein subtypes CLDN18.1 and CLDN18.2 which merely vary in the N-terminal sequences. CLDN18.2 has currently become a very potential action target for anti-tumor drugs due to the expression specificity in tumor cells and normal tissues. WO 2016165762 A1 discloses an anti-CLDN18.2 antibody IMAB362 (Zolbetuximab), which significantly prolongs survival in a phase II clinical trial of gastric cancer relative to standard chemotherapy (13.2 months vs. 8.4 months) and shows a clear advantage in patients with high CLDN18.2 expression.

WO 2021003082 A1 discloses an anti-CD47/anti-CLDN18.2 bispecific antibody, which essentially does not bind to human red blood cells. However, there remains a need to develop a new bispecific antibody targeting CLDN18.2 and CD47 to provide more possibilities for cancer treatment.

### Summary of the Invention

In one aspect, the present invention provides a bispecific antibody comprising a first antigen binding portion that binds to CD47 and a second antigen binding portion that binds to CLDN18.2, wherein the first antigen binding portion comprises a heavy chain variable region (VH) and a light chain variable region (VL), the heavy chain variable region comprises: 1) HCDR1 comprising the amino acid sequence of SEQ ID NO: 4; 2) HCDR2 comprising the amino acid sequence of SEQ ID NO: 5; and 3) HCDR3 comprising the amino acid sequence of SEQ ID NO: 6; and the light chain variable region comprises: 1) LCDR1 comprising the amino acid sequence of SEQ ID NO: 7; 2) LCDR2 comprising the amino acid sequence of SEQ ID NO: 8; and 3) LCDR3 comprising the amino acid sequence of SEQ ID NO: 9.

In an embodiment, the second antigen binding portion comprises an immunoglobulin single variable domain (VHH) that binds to CLDN18.2. Preferably, the immunoglobulin single variable domain comprises: CDR1 comprising the amino acid sequence of SEQ ID NO: 13; CDR2 comprising the amino acid sequence of SEQ ID NO: 14; and CDR3 comprising the amino acid sequence of SEQ ID NO: 15.

In yet another aspect, the present invention provides an isolated polynucleotide encoding the bispecific antibody of the present invention.

The present invention also provides an expression vector comprising the polynucleotide of the present invention.

In another aspect, the present invention provides a host cell comprising the polynucleotide or expression vector of the present invention.

The present invention also relates to an antibody conjugate comprising the bispecific antibody of the present invention conjugated with at least one therapeutic agent.

In yet another aspect, the present invention relates to a pharmaceutical composition comprising the bispecific antibody or the antibody conjugate of the present invention, and a pharmaceutically acceptable carrier.

The present invention also relates to the use of the bispecific antibody, the antibody conjugate, or the pharmaceutical composition of the present invention in the preparation of a drug for treating cancer.

### Brief Description of the Drawings

Fig. 1 shows a schematic structure of the anti-CD47/anti-CLDN18.2 bispecific antibody.
Figs. 2a and 2b show the binding activity of the anti-CD47/anti-CLDN18.2 bispecific antibody to CD47 on red blood cells.
Figs. 3a and 3b show the binding activity of the anti-CD47/anti-CLDN18.2 bispecific antibody on tumor cells expressing a single target and double targets, wherein Fig. 3a shows the binding activity of the anti-CD47/anti-CLDN18.2 bispecific antibody on NUGC-4 cells, and Fig. 3b shows the binding activity of the anti-CD47/anti-CLDN18.2 bispecific antibody on hCLDN18.2-NUGC-4 cells.
Figs. 4a, 4b and 4c show the ability of the anti-CD47/anti-CLDN18.2 bispecific antibody to block the binding of human CD47 to the receptor SIRPα on tumor cells expressing a single target and double targets, wherein Fig. 4a shows the ability of the anti-CD47/anti-CLDN18.2 bispecific antibody to block the binding of human CD47 to the receptor SIRPα on NUGC-4 cells, and Figs. 4b and 4c show the ability of the anti-CD47/anti-CLDN18.2 bispecific antibody to block the binding of human CD47 to the receptor SIRPα on hCLDN18.2-NUGC-4 cells.
Figs. 5a and 5b show the inhibitory effects of the anti-CD47/anti-CLDN18.2 bispecific antibody on tumor growth in mice.
Fig. 6 shows the ADCP activity of the anti-CD47/anti-CLDN18.2 bispecific antibody determined by the bioluminescent reporter gene method.

### Detailed Description of Embodiments

### Definitions

In the present invention, all scientific and technical terms used herein have the meanings typically understood by a person skilled in the art unless specified otherwise. In addition, the terms and laboratory operation steps related to the protein and nucleic acid chemistry, molecular biology, cell and tissue culture, microbiology and immunology used herein are terms and conventional steps that are widely used in the corresponding art. To better understand the present invention, definitions and explanations of related terms are provided below.

As used herein, the expressions "including", "comprising", "containing" and "having" are open-ended and indicate that the listed elements, steps or components are included, but additional unlisted elements, steps or components are not excluded. The expression "consisting of" does not include any unspecified element, step or component. The expression "consisting essentially of" means that the range is limited to the specified elements, steps or components, and optionally, elements, steps or components that do not significantly affect the basic and novel features of the claimed subject matter. It should be understood that the expressions "consisting essentially of" and "consisting of" are encompassed in the meaning of the expression "including".

As used herein, an "antibody" refers to an immunoglobulin or a fragment thereof, which specifically binds to an antigen epitope through at least one antigen binding site. The definition of the antibody herein encompasses an antigen binding fragment. The term "antibody" includes a multispecific antibody (for example, a bispecific antibody), a human antibody, a non-human antibody, a humanized antibody, a chimeric antibody, a single domain antibody, and an antigen binding fragment. The antibody may be synthetic (e.g., produced by chemical or biological coupling), enzymatically treated, or recombinant. The antibody provided herein includes any immunoglobulin type (e.g., IgG, IgM, IgD, IgE, IgA, and IgY), class (e.g., IgG1, IgG2, IgG3, IgG4, IgA1, and IgA2), or subclass (e.g., IgG2a and IgG2b). The antibody may be "univalent", "bivalent", "trivalent" or "tetravalent" antibodies or antibodies with higher valency, which means that the antibody comprises 1, 2, 3, 4 or more antigen binding sites.

As used herein, a "full-length antibody" typically comprises four polypeptides: two heavy chains (HC) and two light chains (LC). Each light chain comprises a "light chain variable region (VL)" and a "light chain constant region (CL)" from the N-terminus (the amino terminus) to the C-terminus (the carboxyl terminus). Each heavy chain contains a "heavy chain variable region (VH)" and a "heavy chain constant region (CH)" from the N-terminus to the C-terminus. In general, the heavy chain constant region of a full-length antibody may comprise CH1-hinge-CH2-CH3 from the N-terminus to the C-terminus. In certain immunoglobulin types (e.g., IgM and IgE), the heavy chain constant region may comprise CH1-hinge-CH2-CH3-CH4 from the N-terminus to the C-terminus.

The light chain variable region and the heavy chain variable region may each comprise three highly variable "complementarity determining regions (CDRs)" and four relatively conserved "framework regions (FRs)", which are connected from the N-terminus to the C-terminus in the order of FR1-CDR1-FR2-CDR2-FR3-CDR3-FR4. The CDR of a light chain variable region (CDRL or LCDR) herein may be referred to as LCDR1, LCDR2 and LCDR3, and the CDR of a heavy chain variable region (CDRH or HCDR) may be referred to as HCDR1, HCDR2 and HCDR3.

In the present invention, the amino acid sequences of CDRs are shown according to the rules of the AbM definition (the sequences in the claims of the present invention are also shown according to the rules of the AbM definition). However, it is well known to those skilled in the art that the CDR of an antibody may be defined by various methods in the art, for example, Chothia based on the three-dimensional structure of antibodies and the topology of CDR loops (see, for example, Chothia, C. et al., Nature, 342, 877-883 (1989); and Al-Lazikani, B. et al., J. Mol. Biol., 273, 927-948 (1997)), Kabat based on antibody sequence variability (see, for example, Kabat, E.A. et al. (1991) Sequences of Proteins of Immunological Interest, Fifth Edition, U.S. Department of Health and Human Services, NIH Publication No. 91-3242), AbM (Martin, A.C.R. and J. Allen (2007) "Bioinformatics tools for antibody engineering" in S. Dübel (ed.), Handbook of Therapeutic Antibodies. Weinheim: Wiley-VCH Verlag, pp. 95-118), Contact (MacCallum, R. M. et al., (1996) J. Mol. Biol. 262:732-745), IMGT (Lefranc, M.-P., 2011 (6), IMGT, the International ImMunoGeneTics Information System Cold Spring Harb Protoc.; and Lefranc, M. - P. et al., Dev. Comp. Immunol., 27, 55-77 (2003)), and North CDR definition based on affinity propagation clustering using a large number of crystal structures. It should be understood by a person skilled in the art that unless otherwise specified, the terms "CDR" and "complementarity determining region" of a given antibody or region thereof (e.g., a variable region) should be understood to encompass a complementarity determining region as defined by any of the above-mentioned known schemes described in the present invention. Although the scope of protection as claimed in the claims of the present invention is based on the sequences shown according to the rules of the AbM definition, corresponding amino acid sequences according to the rules of other CDR definitions shall also fall within the scope of protection of the present invention.

Therefore, when it comes to defining an antibody with specific CDR sequences defined in the present invention, the scope of the antibody also encompasses such an antibody whose variable region sequences comprise the specific CDR sequences but whose claimed CDR boundaries are different from the specific CDR boundaries defined by the present invention due to the application of a different scheme (for example, rules of different assignment systems or their combinations).

As used herein, the terms "framework region" and "FR" may be used interchangeably. As used herein, the term "framework region" residue or "FR" residue refers to those amino acid residues in the variable region of the antibody other than the CDR sequences defined above.

As used herein, a "single domain antibody (sdAb)" or a "nanobody" refers to an antibody comprising a single immunoglobulin variable domain (single variable domain) as a functional antigen binding fragment. Similar to the variable region of a full-length antibody, a single variable domain typically comprises CDR1, CDR2 and CDR3 that form an antigen binding site and a supporting framework region. The single variable domain may be, for example, a variable domain of a heavy chain antibody (VHH), a shark IgNAR variable domain, a human light chain antibody variable domain and a human heavy chain antibody variable domain.

As used herein, the term "antibody dependent cell-mediated phagocytosis" or "ADCP" refers to a cell-mediated process in which non-specific cytotoxic cells (for example, monocytes, macrophages, neutrophils, and dendritic cells) expressing Fc γ receptors (FcyR) recognize antibodies that bind to target cells (such as tumor cells), and then phagocytose target cells (such as tumor cells) as effector cells. In some embodiments, the anti-CD47/anti-CLDN18.2 bispecific antibody of the present invention mediates the ADCP targeting cancer cells expressing CLDN18.2, in particular cancer cells expressing CD47 and CLDN18.2.

As used herein, the terms "percent (%) sequence identity" and "sequence identity" of amino acid sequences have a well-recognized meaning in the art and refer to the percentage of the identical parts of two polypeptide sequences determined by sequence alignment (for example, by manual inspection or a well-known algorithm). The percentage can be determined using methods known to a person skilled in the art, for example, using an available computer software such as BLAST, BLAST-2, Clustal Omega and FASTA software.

An amino acid sequence "originated from" or "derived from" a reference amino acid sequence herein is partially or completely identical or homologous to the reference amino acid sequence. For example, an amino acid sequence derived from a human immunoglobulin heavy chain constant region may have at least 80%, at least 85%, at least 90%, at least 91%, at least 92%, at least 93%, at least 94%, at least 95%, at least 96%, at least 97%, at least 98%, at least 99% or 100% sequence identity to the wild-type sequence of the human immunoglobulin heavy chain constant region from which the amino acid sequence is originated.

Non-critical regions in a polypeptide (for example, CDR regions of an antibody, non-critical amino acids of framework regions, amino acids of constant regions) can be modified, for example, by making substitution, addition and/or deletion of one or more amino acids without altering the function of the polypeptide. A person skilled in the art should understand that amino acids in the non-critical regions of a polypeptide can be substituted with suitable conservative amino acids while retaining its biological activity as a general rule (see, for example, Watson et al., Molecular Biology of the Gene, 4th Edition, 1987, The Benjamin/Cummings Pub. co., p. 224). Suitable conservative substitution is well known to a person skilled in the art. In some cases, the amino acid substitution is a non-conservative substitution. A person skilled in the art should understand that the performance of an antibody or an antibody fragment may be altered by making amino acid mutation or modification, for example, the antibody glycosylation type and the ability to form an interchain disulfide bond may be altered, or an active group may be provided for the preparation of an antibody conjugate. Antibodies or antigen binding fragments thereof containing such amino acid mutations or modifications are also encompassed in the scope of the bispecific antibody of the present invention.

The anti-CD47/anti-CLDN18.2 bispecific antibody or the polynucleotide encoding same according to the present invention may be isolated. As used herein, the expression "isolated" means that a substance (for example, a polynucleotide or a polypeptide) is isolated from the source or environment in which the substance exists, that is, the substance is substantially free of any other ingredients.

The terms "polynucleotide" and "nucleic acid" herein may be interchangeably used to represent an oligomer or a polymer comprising at least two linked nucleotides or nucleotide derivatives and may typically include deoxyribonucleic acid (DNA) and ribonucleic acid (RNA).

The term "vector" herein is a vehicle used to introduce a foreign polynucleotide into a host cell. When the vector is transformed into an appropriate host cell, the foreign polynucleotide can be amplified or expressed. The vector typically remains free, but may be designed to integrate the gene or a part thereof into the chromosome of the genome. As used herein, the definition of a "vector" encompasses a plasmid, a linearized plasmid, a viral vector, a cosmid, a phage vector, a phagemid, an artificial chromosome (for example, a yeast artificial chromosome and a mammalian artificial chromosome), etc. The viral vector includes but is not limited to a retrovirus vector (including a lentiviral vector), an adenovirus vector, an adeno-associated virus vector, a herpes virus vector, a poxvirus vector, a baculovirus vector, etc.

As used herein, the term "expression" refers to the production of an RNA and/or a polypeptide.

As used herein, the term "expression vector" refers to a vector capable of expressing a polynucleotide of interest (including DNA and RNA). For example, in an expression vector, a polynucleotide sequence (including DNA and RNA) encoding a polypeptide of interest may be operably linked to a regulatory sequence (such as a promoter and a ribosome binding site) that can affect the expression of the polynucleotide sequence. The regulatory sequence may comprise promoter and terminator sequences, and may optionally comprise an origin of replication, a selectable marker, an enhancer, a polyadenylation signal, etc. The expression vector may be a plasmid, a phage vector, a recombinant virus or other vector which results in the expression of a polynucleotides of interest upon introduction into an appropriate host cell. Suitable expression vectors are well known to a person skilled in the art. A person skilled in the art can prepare the expression vector as a vector that is replicable in a host cell, remains free in a host cell, or is integrated into the genome of a host cell genome as required.

As used herein, a "host cell" refers to a cell used to receive, maintain, replicate, or amplify a vector. The host cell may also be used to express a polypeptide encoded by a polynucleotide or a vector. The host cell may be a eukaryotic cell or a prokaryotic cell. The prokaryotic cell is e.g., *E. coli* or *Bacillus subtilis,* and the eukaryotic cell is e.g., a yeast cell or an *Aspergillus* cell, an insect cell (such as a *Drosophila* S2 cell or an Sf9 cell), and an animal cell (such as a fibroblast, a CHO cell, a COS cell, a HeLa cell, a NSO cell, or a HEK293 cell).

As used herein, the term "treating" refers to ameliorating diseases/symptoms, for example, reducing or eliminating the diseases/symptoms, or preventing or slowing the occurrence, progression and/or deterioration of the diseases/symptoms. Therefore, the term "treating" includes preventing, treating, and/or curing.

As used herein, the term "pharmaceutically acceptable carrier" refers to a carrier that is pharmacologically and/or physiologically compatible with the subject and the active ingredient and that is well known in the art (see, for example, Remington's Pharmaceutical Sciences. edited by Gennaro AR, 19th ed. Pennsylvania: Mack Publishing Company, 1995), including but not limited to: a pH adjusting agent, a surfactant, an adjuvant, an ion strength enhancer, a diluent, a reagent for maintaining the osmotic pressure, an absorption delaying agent, and a preservative. For example, the pH adjusting agent includes but is not limited to a phosphate buffer. The surfactant includes but is not limited to cationic, anionic or nonionic surfactants, such as Tween-80. The ion strength enhancer includes but is not limited to sodium chloride. The preservative includes but is not limited to various antibacterial reagents and antifungal reagents, such as parabens, chlorobutanol, phenol, and sorbic acid. The reagent for maintaining the osmotic pressure includes but is not limited to sugar, NaCl, and an analog thereof. The absorption delaying agent includes but is not limited to monostearate and gelatin. The diluent includes but is not limited to water, aqueous buffers (such as buffered saline), alcohols, polyols (such as glycerol), etc. The preservative includes but is not limited to various antibacterial reagents and antifungal reagents, such as thiomersal, 2-phenoxyethanol, parabens, chlorobutanol, phenol, and sorbic acid. A "stabilizer" has the meaning that is commonly understood by a person skilled in the art and can stabilize the desired activity of the active ingredient in a drug. The stabilizer includes but is not limited to sodium glutamate, gelatin, SPGA (Sucrose-Phosphate-Glutamate-Albumin), sugars (such as sorbitol, mannitol, starch, sucrose, lactose, dextran or glucose), amino acids (such as glutamic acid or glycine), proteins (such as dry whey, albumin or casein), or degradation products thereof (such as lactalbumin hydrolysate), etc.

As used herein, examples of mammals include but are not limited to human, non-human primate, rat, mouse, cattle, horse, pig, sheep, alpaca, dog, cat, etc. The term "subject" herein refers to a mammal, for example, a human. In some embodiments, the subject is a human. In some embodiments, the subject is a cancer patient, a human or an animal suspected of having cancer or at risk of having cancer.

### Anti-CD47/anti-CLDN18.2 bispecific antibody

The present invention provides an anti-CD47/anti-CLDN18.2 bispecific antibody comprising a first antigen binding portion that binds to CD47 and a second antigen binding portion that binds to CLDN18.2, wherein the first antigen binding portion comprises a heavy chain variable region (VH) and a light chain variable region (VL),
the heavy chain variable region comprises:
   1) HCDR1 comprising the amino acid sequence of SEQ ID NO: 4 or a variant thereof;
   2) HCDR2 comprising the amino acid sequence of SEQ ID NO: 5 or a variant thereof; and
   3) HCDR3 comprising the amino acid sequence of SEQ ID NO: 6 or a variant thereof; and
the light chain variable region comprises:
   1) LCDR1 comprising the amino acid sequence of SEQ ID NO: 7 or a variant thereof;
   2) LCDR2 comprising the amino acid sequence of SEQ ID NO: 8 or a variant thereof; and
   3) LCDR3 comprising the amino acid sequence of SEQ ID NO: 9 or a variant thereof,
wherein the variant has substitution, addition and/or deletion of one or two amino acids compared with the sequence from which the variant is originated.

In a specific embodiment, the first antigen binding portion comprises a first antigen binding portion that specifically binds to CD47, and the first antigen binding portion comprises a heavy chain variable region (VH) and a light chain variable region (VL),
the heavy chain variable region comprises:
   1) HCDR1 comprising the amino acid sequence of SEQ ID NO: 4;
   2) HCDR2 comprising the amino acid sequence of SEQ ID NO: 5; and
   3) HCDR3 comprising the amino acid sequence of SEQ ID NO: 6; and
the light chain variable region comprises:
   1) LCDR1 comprising the amino acid sequence of SEQ ID NO: 7;
   2) LCDR2 comprising the amino acid sequence of SEQ ID NO: 8; and
   3) LCDR3 comprising the amino acid sequence of SEQ ID NO: 9.

In an embodiment, the VH comprises: 1) the amino acid sequence of SEQ ID NO: 10; or 2) an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO: 10; and/or
the VL comprises: 1) the amino acid sequence of SEQ ID NO: 11; or 2) an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO: 11.

In an embodiment, the VH comprises the amino acid sequence of SEQ ID NO: 10, and the VL comprises the amino acid sequence of SEQ ID NO: 11.

The first antigen binding portion may comprise any form of an antigen binding fragment, for example, scFv, dsFv, scdsFv, Fab, Fab' or F(ab')₂. According to the present invention, the first antigen binding portion specifically binds to CD47 on the surface of cancer cells, but does not or substantially does not bind to CD47 on red blood cells, so that the bispecific antibody of the present invention does not cause red blood cell agglutination.

The second antigen binding portion may comprise any form of an antigen binding fragment, including but not limited to scFv, dsFv, scdsFv, Fab, Fab', F(ab')₂, and a single variable domain. In some embodiments, the second antigen binding portion comprises an immunoglobulin single variable domain that specifically binds to CLD18.2. An immunoglobulin single variable domain specifically binding to CLD18.2 is described in for example, CN 112480248 A and WO 2020238730 A1, the contents of which are incorporated herein by reference in their entireties. In an embodiment, the immunoglobulin single variable domain comprises: CDR1 comprising the amino acid sequence of SEQ ID NO: 13 or a variant thereof; CDR2 comprising the amino acid sequence of SEQ ID NO: 14 or a variant thereof; and CDR3 comprising the amino acid sequence of SEQ ID NO: 15 or a variant thereof, wherein the variant has substitution, addition and/or deletion of one or two amino acids compared with the sequence from which the variant is originated. In a specific embodiment, the immunoglobulin single variable domain comprises: CDR1 comprising the amino acid sequence of SEQ ID NO: 13; CDR2 comprising the amino acid sequence of SEQ ID NO: 14; and CDR3 comprising the amino acid sequence of SEQ ID NO: 15. In an embodiment, the immunoglobulin single variable domain comprises the amino acid sequence of SEQ ID NO: 12. In yet another embodiment, the immunoglobulin single variable domain comprises an amino acid sequence having at least 80%, at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 12.

In some embodiments, the first antigen binding portion and the second antigen binding portion are connected through a linker. The linker may be a peptide linker or a chemical bond, preferably a peptide linker. An exemplary peptide linker may include but is not limited to polyglycine (G), polyalanine (A), polyserine (S), or a combination thereof, for example, GGAS, GGGS, GGGSG, or (G¬₄S)ₙ, wherein n is an integer of 1-20. Preferably, n is an integer of 1-5. In a specific embodiment, the peptide linker comprises the amino acid sequence of SEQ ID NO: 22 or SEQ ID NO: 23.

In some embodiments, the anti-CD47/anti-CLDN18.2 bispecific antibody of the present invention further comprises an immunoglobulin constant region. The immunoglobulin constant region may be a heavy chain constant region (CH) and a light chain constant region (CL) of an immunoglobulin of any species. The heavy chain constant region can be derived from a heavy chain constant region of an immunoglobulin of any subtype (e.g., IgA, IgD, IgE, IgG and IgM), class (e.g., IgG1, IgG2, IgG3, IgG4, IgA1 and IgA2), or subclass (e.g., IgG2a and IgG2b) or a combination thereof. In a preferred embodiment, the heavy chain constant region at least comprises an Fc region, for example, the heavy chain constant region of IgG1 may comprise: all or part of the hinge region-CH2-CH3 or CH1-hinge-CH2-CH3. The light chain constant region may be derived from a λ (Lambda) light chain constant region or κ (Kappa) light chain constant region. In a preferred embodiment, the heavy chain constant region is a human IgG1 heavy chain constant region. In an embodiment, the heavy chain constant region comprises the amino acid sequence of SEQ ID NO: 2. In a preferred embodiment, the light chain constant region is a human kappa light chain constant region. In an embodiment, the light chain constant region comprises the amino acid sequence of SEQ ID NO: 3.

In an embodiment, the VH and the VL of the first antigen binding portion are respectively fused to the N-terminus of the heavy chain constant region and the light chain constant region, and the single variable domain of the second antigen binding portion is optionally fused to the N-terminus of the VH, the N-terminus of the VL, the C-terminus of the heavy chain constant region or the C-terminus of the light chain constant region through a linker. In some embodiments, the anti-CD47/anti-CLDN18.2 bispecific antibody comprises a first polypeptide and a second polypeptide, wherein the first polypeptide comprises the VH and the heavy chain constant region of the first antigen binding portion, the second polypeptide comprises the VL and the light chain constant region of the first antigen binding portion, and the single variable domain of the second antigen binding portion is optionally fused to the N-terminus of the VH or the VL or the C-terminus of the heavy chain constant region or the light chain constant region through a linker. In an embodiment, the single variable domain is optionally fused to the N-terminus of the VH or the C-terminus of the heavy chain constant region through a linker, and the first polypeptide may also be referred to as a fused heavy chain. In an embodiment, the fused heavy chain has the structure of formula (I) or formula (III) as described below. In another embodiment, the single variable domain of the second antigen binding portion is optionally fused to the N-terminus of the VL or the C-terminus of the light chain constant region through a linker, and the second polypeptide may also be referred to as a fused light chain. In an embodiment, the fused light chain has the structure of formula (IV) or formula (VI) as described below.

In an embodiment, the first polypeptide has the structure of formula (I):

VH-CH-Linker-VHH Formula (I),

the second polypeptide has the structure of formula (II):

VL-CL Formula (II),

wherein
the VH and the VL are respectively the heavy chain variable region and the light chain variable region of the first antigen binding portion as described above;
the VHH is the immunoglobulin single variable domain as described above;
the CH and the CL are respectively the heavy chain constant region and the light chain constant region as described above; and
the Linker is a linker.

In an embodiment, the first polypeptide has the structure of formula (I) and comprises the amino acid sequence of SEQ ID NO: 20; and the second polypeptide has the structure of formula (II) and comprises the amino acid sequence of SEQ ID NO: 17.

In an embodiment, the first polypeptide has the structure of formula (III):

VHH-Linker-VH-CH Formula (III),

and the second polypeptide has the structure of formula (II), wherein
the VHH is the immunoglobulin single variable domain as described above;
the VH is the heavy chain variable region of the first antigen binding portion as described above;
the CH is the heavy chain constant region as described above;
formula (II) is as defined above; and
the Linker is a linker.

In an embodiment, the first polypeptide has the structure of formula (III) and comprises the amino acid sequence of SEQ ID NO: 16; and the second polypeptide has the structure of formula (II) and comprises the amino acid sequence of SEQ ID NO: 17.

In an embodiment, the first polypeptide has the structure of formula (III) and comprises the amino acid sequence of SEQ ID NO: 21; and the second polypeptide has the structure of formula (II) and comprises the amino acid sequence of SEQ ID NO: 17.

In yet another embodiment, the first polypeptide has the structure of formula (III), and the second polypeptide has the structure of formula (IV):

VHH-Linker-VL-CL Formula (IV),

wherein
formula (III) is as defined above;
the VHH is the immunoglobulin single variable domain as described above;
the VL is the light chain variable region of the first antigen binding portion as described above;
the CL is the light chain constant region as described above; and
the Linker is a linker.

In an embodiment, the first polypeptide has the structure of formula (III) and comprises the amino acid sequence of SEQ ID NO: 16; and the second polypeptide has the structure of formula (IV) and comprises the amino acid sequence of SEQ ID NO: 19.

In another embodiment, the first polypeptide has the structure of formula (V):

VH-CH Formula (V),

and the second polypeptide has the structure of formula (IV),
wherein
formula (IV) is as defined above;
the VH is the heavy chain variable region of the first antigen binding portion as described above;
the CH is the heavy chain constant region as described above; and
the Linker is a linker.

In an embodiment, the first polypeptide has the structure of formula (V) and comprises the amino acid sequence of SEQ ID NO: 18; and the second polypeptide has the structure of formula (IV) and comprises the amino acid sequence of SEQ ID NO: 19.

In another embodiment, the first polypeptide has the structure of formula (V), and the second polypeptide has the structure of formula (VI):

VL-CL-Linker-VHH Formula (VI),

wherein
formula (V) is as defined above;
the VHH is the immunoglobulin single variable domain as described above;
the VL is the light chain variable region of the first antigen binding portion as described above;
the CL is the light chain constant region as described above; and
the Linker is a linker.

In some embodiments, the anti-CD47/anti-CLDN18.2 bispecific antibody of the present invention can:
1) block the binding of CD47 on the surface of cancer cells to SIRPα;
2) induce macrophages to phagocytose cancer cells expressing CD47 and CLDN18.2; and/or
3) bind to cancer cells expressing CD47 and CLDN18.2 but does not or substantially does not bind to red blood cells.

### Polynucleotide, vector and host cell

In another aspect, the present invention provides an isolated polynucleotide, comprising a polynucleotide sequence encoding the anti-CD47/anti-CLDN18.2 bispecific antibody of the present invention.

The polynucleotide of the present invention can be obtained by using the methods known in the art, for example, by isolating from a phage display library, a yeast display library, an immunized animal, and an immortalized cell (for example, mouse B-cell hybridoma cells, EBV mediated immortalized B cells) or by chemical synthesis. The polynucleotide of the present invention can be codon optimized for a host cell for expression.

In yet another aspect, the present invention also provides a vector comprising the polynucleotide of the present invention. In some embodiments, the polynucleotide of the present invention is cloned into an expression vector. The expression vector may further comprise an additional polynucleotide sequence, for example, a regulatory sequence and an antibiotic resistance gene. The expression vector may also comprise a polynucleotide sequence encoding an additional polypeptide. The additional polypeptide can be, for example, a polypeptide that facilitates the detection and/or isolation of an antibody or an antigen binding fragment, including but not limited to an affinity tag (e.g., a polyhistidine tag (His₆) or a glutathione S-transferase (GST) tag), a polypeptide comprising a protease cleavage site, and a reporter protein (e.g., a fluorescent protein).

In an embodiment, the polynucleotide of the present invention is prepared as a recombinant nucleic acid. The recombinant nucleic acid can be prepared using techniques well known in the art, such as chemical synthesis, recombinant DNA technology (for example, polymerase chain reaction (PCR) technology), etc. (see Sambrook, J., E. F. Fritsch, and T. Maniatis. (1989). Molecular cloning: a laboratory manual, 2nd ed. Cold Spring Harbor Laboratory, Cold Spring Harbor, N.Y.).

The polynucleotide of the present invention may be present in one or more expression vectors. In some embodiments, the expression vector is a DNA plasmid, for example, a DNA plasmid for expression in a bacterial cell, a yeast cell, or a mammalian cell. In some further embodiments, the expression vector is a viral vector. In other embodiments, the expression vector is a phage vector or a phagemid vector.

The present invention also provides a host cell comprising at least one polynucleotide or vector as described above. The polynucleotide or expression vector of the present invention can be introduced into a suitable host cell by using various methods known in the art. Such methods include but are not limited to lipofection, electroporation, viral transduction, calcium phosphate transfection, etc.

In a preferred embodiment, the host cell is used to express the anti-CD47/anti-CLDN18.2 bispecific antibody of the present invention. Examples of the host cell include but are not limited to a prokaryotic cell (such as, bacteria, e.g., *E. coli*) and a eukaryotic cell (such as, yeast, an insect cell, and a mammalian cell).

A mammalian host cell suitable for antibody expression includes but is not limited to a myeloma cell, a HeLa cell, a HEK cell (for example, a HEK 293 cell), a Chinese hamster ovary (CHO) cell, and other mammalian cells suitable for antibody expression.

The present invention also provides a method for producing the anti-CD47/anti-CLDN18.2 bispecific antibody of the present invention, which method comprises the following steps:
(I) cultivating the host cell of the present invention under suitable conditions to express the anti-CD47/anti-CLDN18.2 bispecific antibody, and
(II) isolating the antibody from the host cell or a culture thereof.

In some embodiments, a single vector comprising polynucleotide sequences encoding a heavy chain and a light chain is used. In some embodiments, two vectors respectively encoding the antibody light chain and the antibody heavy chain are used. In some embodiments, the host cell also comprises a chaperone plasmid, which may help to improve the solubility, stability, and/or folding of the antibody. Techniques for isolating and purifying an antibody from a host cell or culture media thereof are well known to a person skilled in the art.

### Antibody conjugate

The present invention also provides an antibody conjugate comprising the anti-CD47/anti-CLDN18.2 bispecific antibody of the present invention conjugated with at least one therapeutic agent. The antibody-drug conjugate (ADC) is a typical antibody conjugate, wherein the therapeutic agent may be, for example, a cytotoxic agent.

As used herein, "conjugation" refers to the interconnection of two or more parts by covalent or non-covalent interactions. In a preferred embodiment, the conjugation is covalent conjugation.

The therapeutic agent can be selected from a cytotoxic agent, a therapeutic antibody (e.g., an antibody or an antigen binding fragment thereof specifically binding to an additional antigen), a radioisotope, an oligonucleotide and an analog thereof (e.g., an interfering RNA), a bioactive peptide, a protein toxin (e.g., diphtheria toxin, ricin) and an enzyme (e.g., urease).

The cytotoxic agent refers to a substance that inhibits or reduces cell activity and function and/or kills the cells. Examples of the cytotoxic agent may include but are not limited to: maytansinoid (e.g., maytansine), auristatins (e.g., MMAF, MMAE, MMAD), duostatin, cryptophycin, vinca alkaloids (e.g., vinblastine, vincristine), colchicines, aplysiatoxins, taxane, taxol, docetaxel, cabazitaxel, enediyne antibiotics, cytochalasins, camptothecins, anthracycline antibiotics (e.g., daunorubicin, dihydroxyanthracindione, doxorubicin), cytotoxic antibiotics (e.g., mitomycin, actinomycin, duocarmycin (e.g., CC-1065), auromycin, duomycin, calicheamicin, endomycin, phenomycin), adriamycin, rubidomycin, calicheamicin, cisplatin, ethidium bromide, bleomycin, mitomycin, mithramycin, pladienolide, podophyllotoxin, etoposide, mitoxantrone, 5-fluorouracil, cytarabine, gemcitabine, mercaptopurine, pentostatin, fludarabine, cladribine, nelarabine, carmustine, lomustine, methotrexate, melphalan, tenoposide, glucocorticoid, etc.

The radioisotope can be selected from, for example, ²¹²Bi, ²¹³Bi, ¹³¹I, ¹²⁵I, ¹¹¹In, ¹⁷⁷Lu, ¹⁸⁶Re, ¹⁸⁸Re, ¹⁵³Sm, and ⁹⁰Y. A radioisotope-labeled antibody may also be referred to as a radioactive immunoconjugate.

In a preferred embodiment, the bioactive polypeptide is a polypeptide or protein having a therapeutic activity, binding activity, or enzyme activity. Non-limiting examples of the bioactive polypeptide may include but are not limited to: a protein toxin (e.g., diphtheria toxin, ricin), an enzyme (e.g., urease, horseradish peroxidase), and a cytokine.

In an embodiment, the therapeutic agent is a molecule having a biological activity against tumors. The molecule having a biological activity against tumors includes but is not limited to a cytotoxic agent, a chemotherapeutic agent, a radioisotope, an immune checkpoint inhibitor, an antibody targeting a tumor-specific antigen and other anti-tumor drugs. In a preferred embodiment, the therapeutic agent is a cytotoxic agent. In yet another preferred embodiment, the therapeutic agent is a radioisotope.

The therapeutic agent can be conjugated with the anti-CD47/anti-CLDN18.2 bispecific antibody of the present invention through a linker using any technique known in the art. The linker may comprise an active group for covalent conjugation, for example, amine, hydroxylamine, a maleimide group, carboxyl, phenyl, mercaptan, sulfhydryl or hydroxyl. The linker may be cleavable or non-cleavable. A cleavable linker is, for example, an enzymatically cleavable linker (e.g., a peptide comprising a protease cleavage site), a pH-sensitive linker (e.g., a hydrazone linker), or a reducible linker (e.g., a disulfide bond).

In an embodiment, the linker comprises an active group selected from amine, hydroxylamine, a maleimide group, carboxyl, phenyl, mercaptan, sulfhydryl and hydroxyl. In an embodiment, the linker is a chemical bond. In an embodiment, the linker comprises an amino acid or a peptide consisting of 2-10 amino acids. The amino acid may be natural or non-natural amino acids.

In some embodiments, the therapeutic agent and the linker are conjugated to form an intermediate prior to conjugation with the anti-CD47/anti-CLDN18.2 bispecific antibody of the present invention. In some embodiments, the intermediate is connected to the anti-CD47/anti-CLDN18.2 bispecific antibody of the present invention by forming a thioether bond with the sulfhydryl group of the bispecific antibody. The structure and preparation method of such intermediates and the method for preparing an antibody conjugate using such intermediates are described in, for example, International Patent Application Publication No. WO 2019114666, the relevant content of which is incorporated herein by reference in its entirety.

### Pharmaceutical composition

The present invention also provides a pharmaceutical composition comprising the anti-CD47/anti-CLDN18.2 bispecific antibody or the antibody conjugate of the present invention, and a pharmaceutically acceptable carrier.

The pharmaceutically acceptable carrier includes but is not limited to: a diluent, a binder and an adhesive, a lubricant, a disintegrant, a preservative, a vehicle, a dispersant, a glidant, a sweetener, a coating, an excipient, a preservative, an antioxidant (such as ascorbic acid, cysteine hydrochloride, sodium bisulfate, sodium pyrosulfite, sodium sulfite, ascorbyl palmitate, butylated hydroxyanisole (BHA), butylated hydroxytoluene (BHT), lecithin, propyl gallate, alpha-tocopherol, citric acid, ethylene diamine tetraacetic acid (EDTA), sorbitol, tartaric acid, phosphoric acid, etc.), a solubilizer, a gelling agent, a softener, a solvent (such as, water, alcohol, acetic acid, and syrup), a buffer (such as, phosphate buffer, histidine buffer, and acetate buffer), a surfactant (for example, a nonionic surfactant, such as polysorbate 80, polysorbate 20, poloxamer or polyethylene glycol), an antibacterial agent, an antifungal agent, an isotonic agent (such as, trehalose, sucrose, mannitol, sorbitol, lactose, and glucose), an absorption retardant, a chelating agent, and an emulsifying agent. For a composition comprising an antibody or an antibody conjugate, a suitable carrier can be selected from a buffer (such as, citrate buffer, acetate buffer, phosphate buffer, histidine buffer, and histidine salt buffer), an isotonic agent (such as, trehalose, sucrose, mannitol, sorbitol, lactose, and glucose), a nonionic surfactant (such as, polysorbate 80, polysorbate 20, and poloxamer), or a combination thereof.

The pharmaceutical compositions provided herein may be in a variety of dosage forms, including but not limited to solid, semi-solid, liquid, powder or freeze-dried forms. Preferably, the pharmaceutical composition is suitable for intravenous, intramuscular, subcutaneous, parenteral, spinal or epidermal administration (such as by injection or infusion). For a composition comprising an antibody or an antibody conjugate, the preferred dosage form may generally be, for example, an injection and a freeze-dried powder.

The pharmaceutical composition provided herein may be administered to a subject by any method known in the art, for example, by systemic or local administration. Administration routes include but are not limited to parenteral (e.g., intravenous, intraperitoneal, intradermal, intramuscular, subcutaneous or intracavitary), topical (e.g., intratumoral), epidural or mucosal (e.g., intranasal, oral, vaginal, rectal, sublingual or topical) administration. It should be understood by a person skilled in the art that the exact administration dose will depend on various factors, for example, the pharmacokinetic properties of a pharmaceutical composition, the duration of the treatment, the excretion rate of a specific compound, the purpose of the treatment, administration routes, and the condition of the subject (e.g., the age, health status, body weight, sex, diet, medical history of the patient), and other factors well-known in the medical field. The administration method may be, for example, injection or infusion.

As a general guide, the administration dose of the anti-CD47/anti-CLDN18.2 bispecific antibody of the present invention may be in the range from about 0.0001 to 100 mg/kg, more typically from 0.01 to 20 mg/kg body weight of the subject. For example, the administration dose may be 0.3 mg/kg body weight, 1 mg/kg body weight, 3 mg/kg body weight, 5 mg/kg body weight, 10 mg/kg body weight or 20 mg/kg body weight, or within the range of 1-20 mg/kg body weight. An exemplary treatment regimen entails administration once a week, once every two weeks, once every three weeks, once every four weeks, once a month, once every three months, once every 3-6 months, or with a short administration interval at the beginning and then an extended interval later. The mode of administration may be intravenous drip.

### Treatment

In yet another aspect, the present invention relates to the use of the anti-CD47/anti-CLDN18.2 bispecific antibody, the antibody conjugate, or the pharmaceutical composition of the present invention in the preparation of a drug for treating a disease in a subject.

The present invention also relates to the anti-CD47/anti-CLDN18.2 bispecific antibody, the antibody conjugate, or the pharmaceutical composition of the present invention for use in the treatment of a disease.

The present invention also provides a method for treating a disease in a subject, which method comprises administering to the subject a therapeutically effective amount of the anti-CD47/anti-CLDN18.2 bispecific antibody, the antibody conjugate, or the pharmaceutical composition of the present invention.

In an embodiment, the disease as described above is cancer. As used herein, the "cancer" includes but is not limited to blood cancer and solid tumor. The cancer can also be a metastatic cancer. "Metastasis" refers to the spread of cancer cells to other parts of the body from their primary site. For example, the anti-CD47/anti-CLDN18.2 bispecific antibody may be used to treat a cancer that is CLDN18.2 positive. In a preferred embodiment, the anti-CD47/anti-CLDN18.2 bispecific antibody is used to treat a cancer that is CD47 and CLDN18.2 positive. In some embodiments, the cancer is gastric cancer.

### Kit

The present invention also provides a kit comprising the anti-CD47/anti-CLDN18.2 bispecific antibody, the antibody conjugate, or the pharmaceutical composition of the present invention, and instructions for use. The kit may also comprise a suitable container. In certain embodiments, the kit also comprises an administration device. In general, the kit also includes a label for indicating the intended use and/or method of use of the contents of the kit. The term "label" includes any written or recorded material provided on or with the kit, or which otherwise accompanies the kit.

### Beneficial effects

The anti-CD47/anti-CLDN18.2 bispecific antibody of the present invention can achieve at least one of the beneficial effects of:
1) blocking the binding of CD47 on the surface of cancer cells to SIRPα;
2) inducing macrophages to phagocytose cancer cells expressing CD47 and CLDN18.2;
3) mediating ADCP targeting the cancer cells expressing CLDN18.2; and
4) binding to cancer cells expressing CD47 and CLDN18.2 while not or substantially not binding to red blood cells.

In addition, compared with monoclonal antibodies targeting CD47 alone, the anti-CD47/anti-CLDN18.2 bispecific antibody of the present invention has a higher binding activity to tumor cells that are CD47 and CLDN18.2 positive, and is more potent in blocking CD47/SIRPα interactions.

### Examples

The examples below are intended to merely illustrate the present invention and should therefore not be considered to limit the present invention in any way.

### Materials and Methods

### 1. Construction of antibody expression plasmid

DNA fragments encoding the antibody heavy chain and the antibody light chain were prepared using the recombinant DNA technology and then respectively cloned into the expression vector pcDNA3.4-TOPO (Invitrogen) to obtain the plasmids for expressing the antibody heavy chain and light chain. The plasmids for expressing the antibody heavy chain and light chain were amplified in *E. coli* DH5α and then extracted and purified.

### 2. Antibody expression and purification

All the antibodies were expressed by using the ExpiCHO transient expression system (Thermo Fisher, A29133) (see WO 2020238730 A1) and affinity purified by using MabSelect SuRe LX (GE, 17547403).

### 3. Antibody purity determination by SDS-PAGE

Preparation of a reducing solution: 2 µg of an antibody to be tested or IPI (Ipilimlumab; reference) was added to 5 × SDS loading buffer (containing DTT at a final concentration of 5 mM). The resulting mixture was heated in a dry bath at 100°C for 10 min, cooled to room temperature, and then centrifuged at 12000 rpm for 5 min, and the supernatant was taken. The supernatant was added to Bis-tris 4-15% gradient gel (Genscript) for protein gel electrophoresis, followed by Coomassie brilliant blue staining for color development of protein bands. After destaining, the protein bands were scanned by EPSON V550 color scanner. The purity was calculated for the protein bands by the peak area normalization method through ImageJ.

### 4. Antibody purity determination by SEC-HPLC

Agilent HPLC 1100 chromatographic column (XBridge BEH SEC 3.5 µm, 7.8 mm I.D. × 30 cm, Waters); the flow rate was set as 0.8 mL/min; injection volume: 20 µL; and detector (VWD) wavelength: 280 nm and 214 nm. Mobile phase: 150 mmol/L phosphate buffer, pH 7.4. All samples were diluted to 0.5 mg/mL with the mobile phase, and then a blank solution and a sample solution were injected in sequence. The percentages of high polymer aggregate, antibody monomer and low molecular aggregate in the sample were calculated according to the area normalization method.

### 5. Antibody stability determination by differential scanning fluorimetry

Differential scanning fluorimetry (DSF) can provide the information about the protein structure stability according to the fluorescence change process in a protein profile, detect the change of the protein configuration, and obtain a melting temperature (Tm) of the protein.

0.2 mg/mL antibody sample solution to be tested was prepared, and PBS and IPI (Ipilimlumab; 0.2 mg/mL) were used as reference. Test sample was added in triplicate at 19 µL/well to a 96-well plate (Nunc), and then 1 µL of 100 × SYPRO orange dye was added to each well. A pipette was used for mixing by pipetting until ready for testing. ABI 7500 FAST RT-PCR instrument was used for the thermal stability test of the sample, melting curve was selected as the test type, and a continuous mode was adopted with the scan temperature ranging 25-95°C at the heating rate of 1%. Equilibration was allowed at 25°C for 5 min and data was collected during the heating process. "ROX" was selected as the reporter group and "None" as the quenching group. The reaction volume was 20 µL. The temperature corresponding to the first peak of the first derivative of the melting curve was determined as the antibody melting temperature Tm.

### Example 1 Design of anti-CD47/anti-CLDN18.2 bispecific antibody

This example describes an exemplary anti-CD47/anti-CLDN18.2 bispecific antibody, wherein the arm that binds to CLDN18.2 adopts the anti-CLDN18.2 single domain antibody NA3S-H1 that specifically recognizes human CLDN18.2 but does not recognize CLDN18.1 (the amino acid sequences of CDR1, CDR2 and CDR3 are respectively set forth in SEQ ID NOs: 13, 14 and 15; and the amino acid sequence of VHH is set forth in SEQ ID NO: 12), and the arm that binds to CD47 adopts the antigen binding domain of the anti-CD47 humanized antibody A7H3L3 (the amino acid sequences of HCDR1, HCDR2 and HCDR3 are respectively set forth in SEQ ID NOs: 4, 5 and 6; the amino acid sequences of LCDR1, LCDR2 and LCDR3 are respectively set forth in SEQ ID NOs: 7, 8 and 9; and the amino acid sequence of the heavy chain variable region is set forth in SEQ ID NO: 10, and the amino acid sequence of the light chain variable region is set forth in SEQ ID NO: 11). The heavy chain variable region of the antibody A7H3L3 was fused to the human IgG1 heavy chain constant region (SEQ ID NO: 2) to form the heavy chain of the antibody A7H3L3, and the light chain variable region was fused to the human kappa light chain constant region (SEQ ID NO: 3) to form the light chain of the antibody A7H3L3. The anti-CLDN18.2 single domain antibody NA3 S-H1 has been published in WO 2020238730 A1 and exhibited excellent efficacies in the *in vitro* cell killing (ADCC and CDC) assay and tumor inhibition trial on human CLDN18.2-HEK29T-SCID tumor transplantation model. The humanized antibody A7H3L3 binds poorly to CD47 on red blood cells and is an ideal candidate antibody for the bispecific antibody. Meanwhile, due to the binding to CLDN18.2, the design of the bispecific antibody enables the binding arm of the anti-CD47 antibody A7H3L3 to better bind to the tumor cells expressing double targets and to better block the SIRPα suppressive signal.

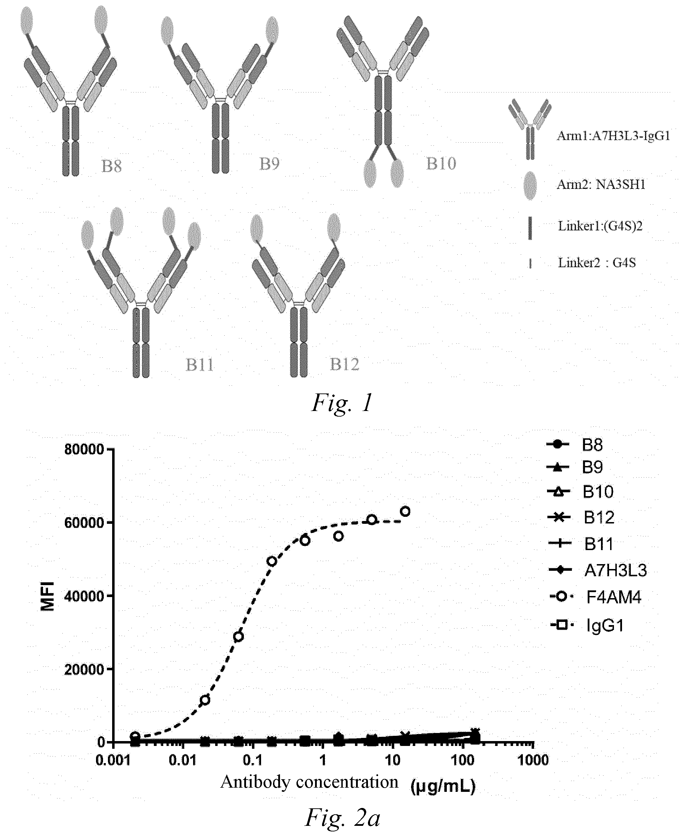

The bispecific antibody was designed according to the valence number, position, and linker length of the anti-CLDN18.2 single domain antibody (VHH) NA3S-H1. Five bispecific antibodies (B8-B12 respectively) were designed. The structures of the exemplary bispecific antibodies are shown in Table 1 and Fig. 1, and the corresponding amino acid sequences are provided in Table 2, wherein the sequence of Linker! is GGGGSGGGGS (SEQ ID NO: 22), and the sequence of Linker2 is GGGGS (SEQ ID NO: 23).

**Table 1 Structure of anti-CD47/anti-CLDN18.2 bispecific antibody**

| Antibody name | Heavy chain structure | Light chain structure |
|---|---|---|
| B8 | VHH^{CLDN18.2}-Linker1-VH^{CD47}-CH1-Hinge-CH2-CH3 | VL^{CD47}-CL |
| B9 | VH^{CD47}-CH1-Hinge-CH2-CH3 | VHH^{CLDN18.2}-Linker1-VL^{CD47}-CL |
| B10 | VH^{CD47}-CH1-Hinge-CH2-CH3-Linker1-VHH^{CLDN18.2} | VL^{CD47}-CL |
| B11 | VHH^{CLDN18.2}-Linker1-VH^{CD47}-CH1-Hinge-CH2-CH3 | VHH^{CLDN18.2}-Linker1-VL^{CD47}-CL |
| B12 | VHH^{CLDN18.2}-Linker2-VH^{CD47}-CH1-Hinge-CH2-CH3 | VL^{CD47}-CL |

**Table 2 Amino acid sequences of anti-CD47/anti-CLDN18.2 bispecific antibody**

| Antibody name | Heavy chain amino acid sequence | Light chain amino acid sequence |
|---|---|---|
| B8 | SEQ ID NO: 16 | SEQ ID NO: 17 |
| B9 | SEQ ID NO: 18 | SEQ ID NO: 19 |
| B10 | SEQ ID NO: 20 | SEQ ID NO: 17 |
| B11 | SEQ ID NO: 16 | SEQ ID NO: 19 |
| B12 | SEQ ID NO: 21 | SEQ ID NO: 17 |

### Example 2 Binding activity of anti-CD47/anti-CLDN18.2 bispecific antibody to CD47 on red blood cells

Flow cytometry was used to determine whether the bispecific antibody was bound to red blood cells. Another anti-CD47 antibody F4AM4-IgG1 (abbreviated as F4AM4 in the accompanying drawings; the amino acid sequence of the heavy chain is SEQ ID NO: 24, and the amino acid sequence of the light chain is SEQ ID NO: 25) developed by the applicant was used as a positive control antibody. F4AM4-IgG1 exhibited strong binding to CD47 of red blood cells in a series of functional validation experiments (conducted prior to the experiment in this example), and hence was selected as a positive control in this example.

The specific method was as follows: red blood cells were isolated from 1 mL of anticoagulant human blood, and the supernatant was aspirated following centrifugation. After the cells were rinsed twice with PBS, 1 mL of PBS was added to resuspend the red blood cells. The red blood cells were diluted to 1 × 10⁷/mL with PBS, pipetted at 50 µL/well and added to a 96-well round bottom cell culture plate. Then an equal volume of the serially diluted antibody to be tested was added, fully mixed, and incubated at 4°C for 1 h. After the culture was rinsed with FACS buffer for three times, 0.5 µg of PE-labeled goat anti-human IgG Fc antibody (Abeam, ab98596) was added, followed by incubation at 4°C for 1 h. Subsequently, the cells were rinsed with FACS buffer for three times and then resuspended by adding 200 µL of FACS buffer, and finally the amount of the antibody binding to the red blood cells (expressed as mean fluorescence intensity (MFI)) was measured by a flow cytometer (Beckman, CytoFLEX AOO-1-1102).

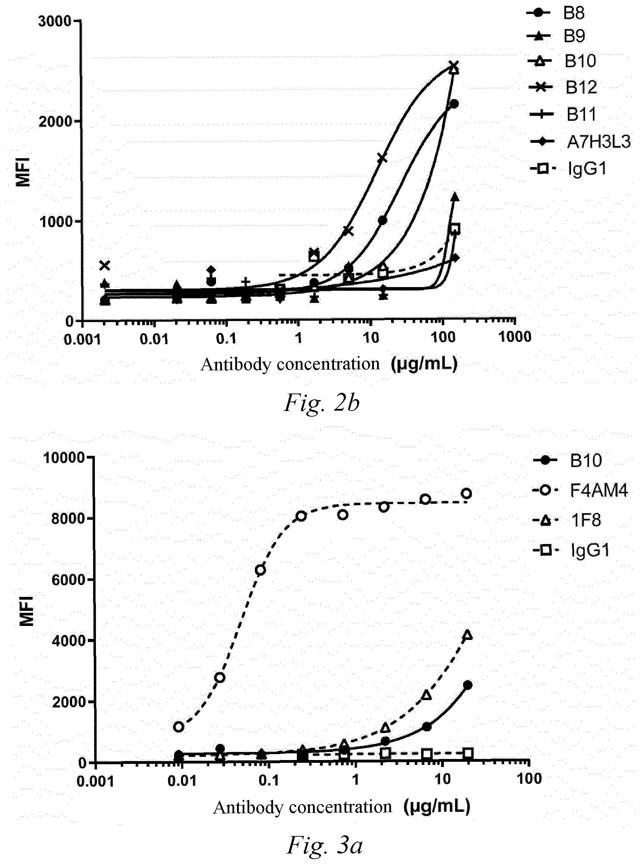

The binding activity of the antibody to red blood cells is shown in Figs. 2a and 2b. As shown in Figs. 2a and 2b, even at a very high concentration (150 µg/mL), almost all the anti-CD47/anti-CLDN18.2 bispecific antibodies did not bind to the red blood cells or had a very low binding activity relative to the strong binding of F4AM4-IgG1 on red blood cells; and at a high concentration (15 µg/mL), there was no significant difference in the binding of red blood cells between B9, B10 and B11 and the negative control IgG1. The specific values are shown in Table 3. It can be seen therefrom that the bispecific antibody of the present invention has a low binding activity to CD47 on red blood cells, and would not substantially cause red blood cell agglutination.

**Table 3 Mean fluorescence intensity (MFI) for binding of bispecific antibody on red blood cells**

| | B8 | B9 | B10 | B11 | B12 | A7H3L3 | IgG1 | F4AM4-IgG1 |
|---|---|---|---|---|---|---|---|---|
| 15 µg/mL MFI | 988 | 239 | 534 | 238 | 1615 | 463 | 303 | 63000 |
| 150 µg/mL MFI | 2145 | 1220 | 2493 | 847 | 2526 | 608 | 901 | > 63000 |

### Example 3 Binding activity of anti-CD47/anti-CLDN18.2 bispecific antibody on tumor cells expressing single target and double targets

The binding activities of the anti-CD47/anti-CLDN18.2 bispecific antibody B10 to NUGC-4 cells (expressing endogenous CD47, purchased from BeNa Culture Collection (BNCC), No. BNCC341962) and hCLDN18.2-NUGC-4 cells (gastric cancer cell line NUGC-4 overexpressing exogenous human CLDN18.2 (amino acid sequence SEQ ID NO: 1) and also expressing endogenous CD47, which cell line was constructed by transfection with lentiviruses) were determined by flow cytometry. As a comparison, the binding activities of the antibody 1F8 (the 1F8 antibody in WO 2018075857 A1) and F4AM4-IgG1 to the two cell lines were also determined. Human IgG1 was used as an isotype negative control.

The specific method was as follows: 1 × 10⁵ NUGC-4 cells or hCLDN18.2-NUGC-4 cells were taken and centrifuged at a low speed (300 g), and the supernatant was removed. The cells at the bottom of the centrifuge tube were rinsed once with a formulated FACS buffer (1×PBS buffer containing 2% by volume of FBS). Subsequently, the serially diluted antibody to be tested was added to the rinsed cells, which were then incubated at 4°C for 1 h. After the culture was rinsed with the above-mentioned FACS buffer for three times, 0.5 µg of PE-labeled goat anti-human IgG Fc antibody (Abcam, ab98596) was added, followed by incubation at 4°C for 1 h. Subsequently, the cells were rinsed with FACS buffer for three times and then resuspended with 200 µL of FACS buffer, and finally the amount of the antibody binding to the red blood cells (expressed as mean fluorescence intensity (MFI)) was measured by a flow cytometer (Beckman, CytoFLEX AOO-1-1102).

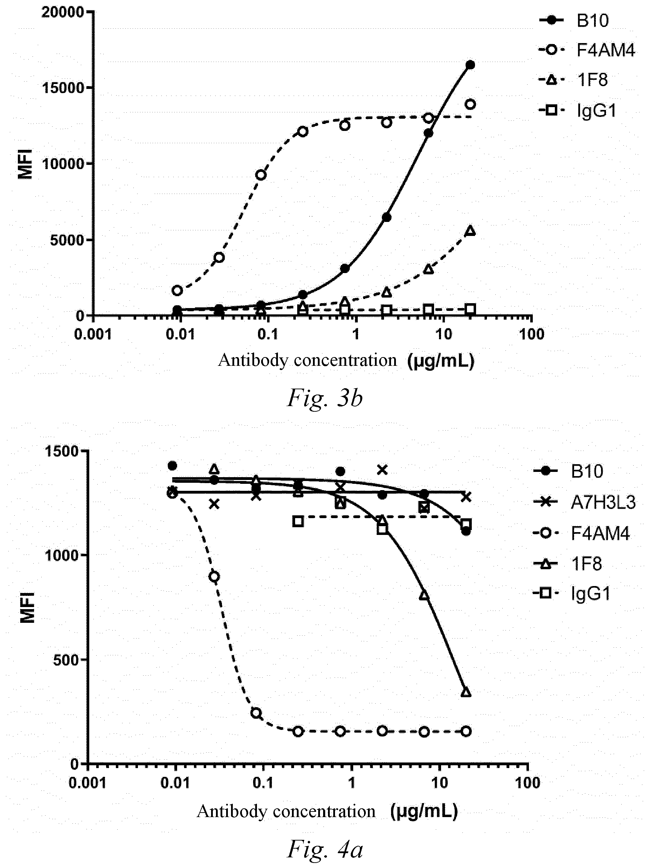

The binding activities of the antibody to the NUGC-4 cells and hCLDN18.2-NUGC-4 cells are respectively shown in Figs. 3a and 3b. As shown in Fig. 3a, the binding activity of the bispecific antibody B10 on the tumor cell NUGC-4 was poor and was slightly weaker than that of the antibody 1F8. As shown in Fig. 3b, the bispecific antibody B10 had a significantly better binding activity on the hCLDN18.2-NUGC-4 cells expressing both CD47 and CLDN18.2 than the antibody 1F8. On this basis, although the binding of the bispecific antibody B10 on cells expressing the single target of CD47 is weaker than that of 1F8, its binding on cells expressing the double targets of CD47 and CLDN18.2 is superior to the binding of 1F8. The results demonstrate that the bispecific antibody B10 can bind to CLDN18.2 and CD47 in the tumor cells at the same time, and has an increased tumor cell-binding ability.

### Example 4 Ability of anti-CD47/anti-CLDN18.2 bispecific antibody to block SIRPα/CD47 binding on tumor cells expressing single target and double targets

Flow cytometry was used to determine the ability of the anti-CD47/anti-CLDN18.2 bispecific antibody to block the binding of CD47 on the NUGC-4 cells and hCLDN18.2-NUGC-4 cells to SIRPα. As a comparison, the ability of the antibodies 1F8, F4AM4-IgG1 and A7H3L3 to block the binding of CD47 on the NUGC-4 tumor cells and hCLDN18.2-NUGC-4 tumor cells to SIRPα was also determined.

The specific method was as follows: 1 × 10⁵ NUGC-4 cells or hCLDN18.2-NUGC-4 cells were taken and centrifuged at a low speed (300 g), and the supernatant was removed. The cells at the bottom of the centrifuge tube were rinsed once with a formulated FACS buffer (1×PBS buffer containing 2% FBS). Subsequently, the serially diluted antibody to be tested was added to the rinsed cells, which were then incubated for 1 h. After the cells were rinsed twice with FACS buffer, 100 µL of 1 µg/mL SIRPα-mFc (ACRO, SIA-H52A8) was added, followed by incubation at 4°C for 1 h. After the cells were rinsed with FACS buffer for three times, 100 µL of 1 : 200 diluted PE-labeled goat anti-mouse Fc (Abcam, ab98742) was added as the secondary antibody, followed by incubation at 4°C for 1 h. After centrifugation, the supernatant was removed, the cells were resuspended by adding 200 µL of FACS buffer, and finally the amount of SIRPα-mFc binding to the cells (expressed as mean fluorescence intensity (MFI)) was measured by a flow cytometer (Beckman, CytoFLEX AOO-1-1102).

The results obtained from the NUGC-4 cells are as shown in Fig. 4a: the antibody F4AM4-IgG1 can effectively block the binding of CD47 and SIRPα with an IC₅₀ of 0.033 µg/mL (0.226 nM); the antibody 1F8 had a weak blocking effect with an IC₅₀ of 15.36 µg/mL (105.6 nM); however, the bispecific antibody B10 had barely any blocking ability.

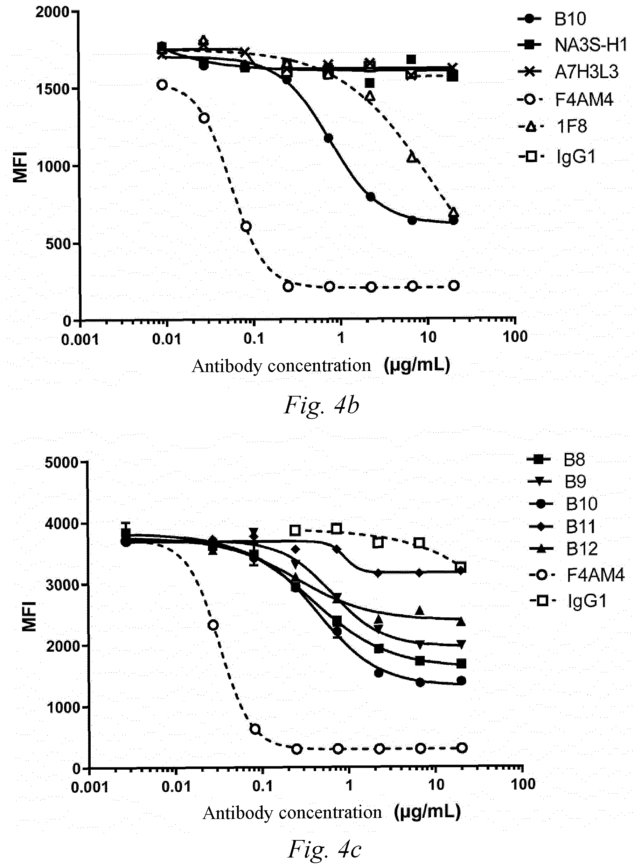

The results obtained from the hCLDN18.2-NUGC-4 cells are as shown in Fig. 4b: the antibody F4AM4-IgG1 also had a strong blocking ability on this tumor cell with an IC₅₀ of 0.056 µg/mL (0.383 nM); compared with the blocking ability on NUGC-4 cells, the blocking ability of the bispecific antibody B10 on the hCLDN18.2-NUGC-4 cells was significantly improved and was superior to that of the antibody 1F8; and B10 and 1F8 blocked the binding of CD47 on hCLDN18.2-NUGC-4 to SIRPα with the IC₅₀ of 0.765 µg/mL (4.476 nM) and 11.98 µg/mL (82.34 nM). Besides, due to the fact that the anti-CLDN18.2 single domain antibody NA3S-H1 was only bound to CLDN18.2, the antibody had no blocking effect. On this basis, although the blocking activity of the bispecific antibody B10 on cells expressing the single target of CD47 is weaker than that of the antibody 1F8, its blocking activity on cells expressing the double targets of CD47 and CLDN18.2 is significantly superior to that of the antibody 1F8 and hence the bispecific antibody has a stronger ability to block the binding of CD47 to the receptor SIRPα.

The blocking ability of other bispecific antibodies on the hCLDN18.2-NUGC-4 cells was also determined. The results are as shown in Fig. 4c. The bispecific antibody B10 among B8-B12 has the highest blocking activity.

### Example 5 In vivo tumor inhibition experiment of anti-CD47/anti-CLDN18.2 bispecific antibody

### 5.1 In vivo tumor inhibition experiment 1

Female nude mice (6-7 weeks old, body weight: 16-18 g) were housed in an independent ventilated box with constant temperature and humidity. The rearing chamber was at the temperature of 21-24°C and the humidity of 30-53%. The nude mice were injected with 3 × 10⁶ hCLDN18.2-NUGC-4 cells subcutaneously in the left armpit (day 0). When the subcutaneous tumor of the mice reached the volume of around 300-400 mm³ (day 20), the mouse samples with too large or too small tumor volumes were culled. Randomized grouping was carried out according to the tumor volume (8 mice per group): PBS treatment group, NA3S-H1 monoclonal antibody administration group, A7H3L3 monoclonal antibody administration group, NA3S-H1 + A7H3L3 co-administration group and bispecific antibody B10 administration group, respectively. With NA3S-H1 monoclonal antibody at 5 mg/kg as the standard, all the other drugs were dosed at equimolar dosages, namely, A7H3L3 monoclonal antibody: 9.4 mg/kg, the combination NA3S-H1 + A7H3L3: 5 mg/kg + 9.4 mg/kg, and the bispecific antibody B10: 10.6 mg/kg. Twice weekly dosing was given by alternate intraperitoneal injection (i.p.) and intravenous injection (i.v.). The length (mm) and width (mm) of the tumor were observed and recorded at any time, and the tumor volume (V) thereof was calculated, using the equations: V = (length × width²)/2, tumor inhibition rate TGI (%) = (1 - average tumor volume in the administration group/average tumor volume in the PBS treatment group) × 100%.

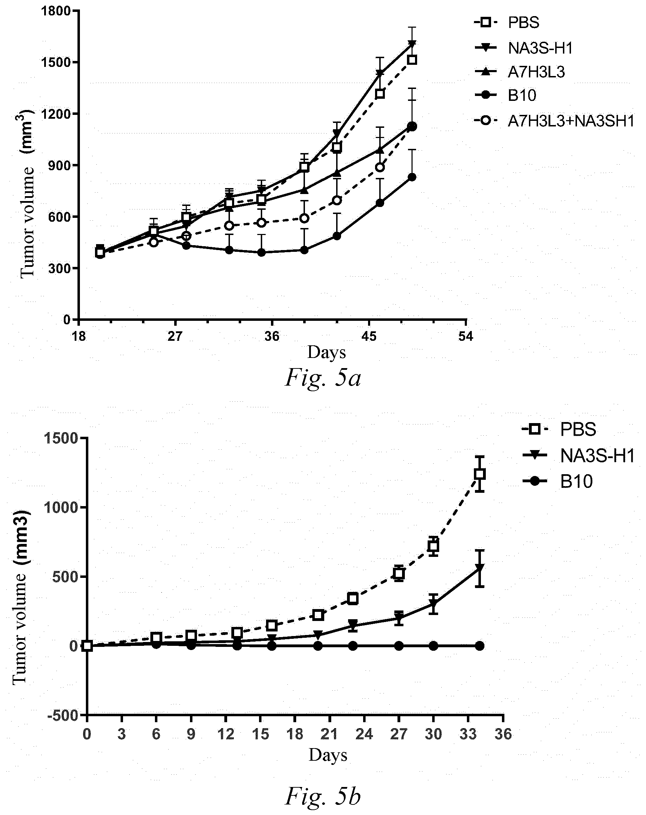

The tumor inhibition results of the antibody are as shown in Fig. 5a and Table 4, from which it can be seen that: at this equimolar dosage, the NA3S-H1 monoclonal antibody administration group exhibited barely any tumor inhibition effect, while the other groups exhibited a certain tumor inhibition effect. The bispecific antibody B10, as the best among them, achieved a tumor inhibition rate close to 54.54% before day 39 with the tumor size close to the initial tumor volume on day 20, which was also superior to the results of the combined administration (A7H3L3 + NA3 S-H1 (9.4 + 5 mpk)). This suggests that the binding of the bispecific antibody B 10 to CLDN18.2 can enhance its blocking effect on the binding of CD47 and SIRPα.

**Table 4 Tumor inhibition rate of bispecific antibody in mice**

| Days | NA3S-H1 | A7H3L3 | B10 | A7H3L3 + NA3S-H1 |
|---|---|---|---|---|
| 25 | 2.96% | -1.31% | 3.41% | 12.73% |
| 28 | 8.76% | 2.17% | 27.69% | 18.40% |
| 32 | -5.33% | 3.73% | 40.26% | 19.30% |
| 35 | -6.94% | 2.10% | 44.31% | 19.49% |
| 39 | 2.01% | 15.00% | 54.54% | 33.71% |
| 42 | -7.26% | 14.65% | 51.45% | 30.84% |
| 46 | -8.69% | 24.65% | 48.33% | 32.59% |
| 49 | -5.94% | 25.18% | 45.12% | 25.62% |

### 5.2 In vivo tumor inhibition experiment 2

Randomized grouping (8 mice per group): PBS treatment group, monoclonal antibody NA3S-H1 administration group, and bispecific antibody B10 administration group, respectively. The first administration was given on the day of inoculation (day 0), followed by a twice weekly dosing regimen. With NA3S-H1 monoclonal antibody at 2.5 mg/kg as the standard, the bispecific antibody B10 was dosed at an equimolar dosage, namely 5.3 mg/kg. The rest was consistent with Example 5.1.

The results are as shown in Fig. 5b. At this equimolar dosage, the NA3S-H1 monoclonal antibody administration group exhibited a certain tumor inhibition effect with a tumor inhibition rate of 54.99% (day 34); and in the bispecific antibody B10 group, complete tumor inhibition was achieved in all the mice and the tumor inhibition rate was nearly 100% (day 34). The results demonstrate that the tumor inhibition effect of the bispecific antibody B10 is significantly superior to that of the anti-CLDN18.2 monoclonal antibody NA3S-H1.

### Example 6 Determination of physicochemical properties of Anti-CD47/anti-CLDN18.2 bispecific antibody

### 6.1 Purity determination by SDS-PAGE

The purity of the bispecific antibody B10 was determined using reducing SDS-PAGE. The apparent relative molecular weights of the main bands of the heavy and light chains of the bispecific antibody B10 were about 65 kD and 25 kD respectively, which were in line with the expected size, and the purity was about 90%.

### 6.2 Purity determination by SEC-HPLC

The monomer purity of the bispecific antibody B10 was determined using SEC-HPLC. The monomer purity of the bispecific antibody B10 determined by SEC-HPLC was greater than 94%.

### 6.3 Thermal stability test of bispecific antibody B10 by DSF

The Tm value of the bispecific antibody B10 was determined using the DSF method to evaluate its thermal stability. The results show that the bispecific antibody B10 had two melting peaks, the Tm value of the first peak was 69.85 ± 0.06°C, and the Tm value of the second peak was 80.60 ± 0.16°C, indicating that the bispecific antibody B10 has a good thermal stability.

### Example 7 ADCP activity of anti-CD47/anti-CLDN18.2 bispecific antibody

Antibody dependent cell-mediated phagocytosis (ADCP) is an important mechanism by which therapeutic antibodies fight against viral infection or tumor cells. In this experiment, the ADCP activity of the bispecific antibody of the present invention was evaluated by the bioluminescent reporter gene method. In this method, genetically engineered Jurkat cells (BPS Bioscience Inc., 71273), which stably express FcyRIIa receptors and are driven by NFAT response elements to express luciferase (Int Immunopharmacol. 2021 Nov; 100:108-112), were used as effector cells. After recognizing the target cell, the antibody activates the NFAT response element in the cell by binding to FcyRIIa on the surface of the effector cell, which in turn drives the expression of luciferase. The activity of luciferase can be quantified by the bioluminescence method.

The samples to be tested (B10, A7H3L3, and NA3S-H1) were diluted to an initial reaction concentration of 200 nM, and 10 dilution gradients were made in 2-fold gradient. The diluted samples were added to a 96-well white bottom plate at 50 µL/well. Three replicate wells were set for each concentration gradient. PBS solution (150 µL/well) was supplemented to the edge wells of the 96-well white bottom plate. Then hCLDN18.2-NUGC-4 cells (target cells, 5 × 10⁵ cells/mL) were added to the 96-well white bottom plate at 50 µL/well and incubated at room temperature for 30 min. Then effector cells as described above (1.5 × 10⁶ cells/mL) were added at 50 µL/well. The 96-well white bottom plate was incubated in a 37°C, 5% CO₂ incubator for 6 h. Detection: the 96-well white bottom plate was taken out and was let to stand for 30 min at room temperature. The detection reagent luciferase substrate (Bio-Glo^{™} Luciferase Assay System Promega G7940) was added at 50 µL/well and allowed to react away from light at room temperature for 5-10 min, and the luminescence intensity (expressed as relative light unit (RLU)) was measured with a microplate reader. The log value of the antibody concentration was plotted against the RLU value at the corresponding concentration, the data was analyzed using Graphpad Prism software, and the EC₅₀ value was calculated.

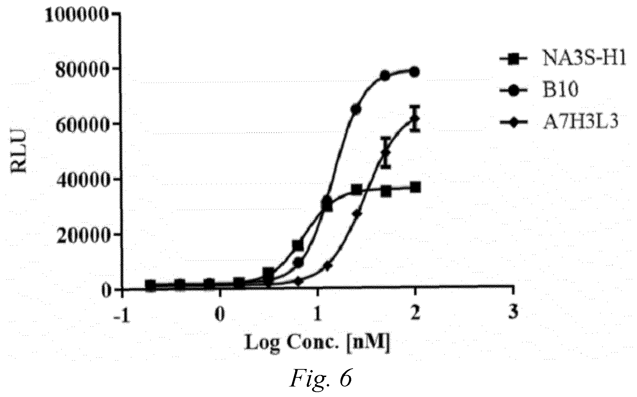

Experimental results: it can be seen from Fig. 6 and Table 5 that when the target cells were hCLDN18.2-NUGC-4 cells, the bispecific antibody B10 of the present invention exhibited a higher ADCP activity compared with the anti-CD47 monoclonal antibody A7H3L3 and the anti-CLDN18.2 monoclonal antibody NA3S-H1.

**Table 5 ADCP activity of bispecific antibody**

| | NA3S-H1 | B10 | A7H3L3 |
|---|---|---|---|
| EC₅₀ | 7.169 | 11.69 | 25.64 |
| R² | 0.9948 | 0.9895 | 0.9962 |

Although the specific embodiments of the present invention have been described in detail, it will be understood by a person skilled in the art that: according to all the teachings disclosed herein, various modifications and changes can be made to the details, and these changes are all within the protection scope of the present invention. The protection scope of the present invention is given by the appended claims and any equivalents thereof.

### Sequence Listing

SEQ ID NO: 1 Human CLDN18.2 
MAVTACQGLGFVVSLIGIAGIIAATCMDQWSTQDLYNNPVTAVFNYQGLWRSCVRE
SSGFTECRGYFTLLGLPAMLQAVRALMIVGIVLGAIGLLVSIFALKCIRIGSMEDSAK
ANMTLTSGIMFIVSGLCAIAGVSVFANMLVTNFWMSTANMYTGMGGMVQTVQTR
YTFGAALFVGWVAGGLTLIGGVMMCIACRGLAPEETNYKAVSYHASGHSVAYKPG
GFKASTGFGSNTKNKKIYDGGARTEDEVQSYPSKHDYV
SEQ ID NO: 2 Human IgG1 heavy chain constant region 
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKAEPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
SEQ ID NO: 3 Human Kapp light chain constant region
SEQ ID NO: 4 A7H3L3-HCDR1
   GFNIKDIYIY
SEQ ID NO: 5 A7H3L3-HCDR2
   KIDPANGNTK
SEQ ID NO: 6 A7H3L3-HCDR3
   GYGSGFAY
SEQ ID NO: 7 A7H3L3-LCDR1
   RASQDISNHLN
SEQ ID NO: 8 A7H3L3-LCDR2
   YTSRIHS
SEQ ID NO: 9 A7H3L3-LCDR3
   QQGYTLPFT
SEQ ID NO: 10 A7H3L3-VH 
QVQLVQSGAEVKKPGASVKVSCKASGFNIKDIYIYWVRQAPGQGLEWIGKIDPANG
NTKYDQKFQGRATITADTSTNTAYLELSSLRSEDTAVYYCARGYGSGFAYWGQGTL
VTVSS
SEQ ID NO: 11 A7H3L3-VL 
DIQMTQSPSSLSASVGDRVTITCRASQDISNHLNWYQQKPGKAPKLLIYYTSRIHSG
VPSSFRGSGSGTDYTLTISSLQPEDIATYFCQQGYTLPFTFGSGTKLEIK
SEQ ID NO: 12 NA3S-H1 
QVQLVESGGGLVQPGGSLRLSCAASGSIFNIPVMGWYRQAPGKQRELVAGISTGGT
TNYGDSVKGRFTISRDNAKNTVYLQMNSLKPEDTAVYYCNVLVVSGIGSTLEVWG
QGTLVTVSS
SEQ ID NO: 13 NA3S-H1 CDR1
   GSIFNIPV
SEQ ID NO: 14 NA3S-H1 CDR2
   ISTGGTT
SEQ ID NO: 15 NA3S-H1 CDR3
   NVLWSGIGSTLEV
SEQ ID NO: 16 B8/B11 heavy chain 
QVQLVESGGGLVQPGGSLRLSCAASGSIFNIPVMGWYRQAPGKQRELVAGISTGGT
TNYGDSVKGRFTISRDNAKNTVYLQMNSLKPEDTAVYYCNVLVVSGIGSTLEVWGQ
GTLVTVSSGGGGSGGGGSQVQLVQSGAEVKKPGASVKVSCKASGFNIKDIYIYWVR
QAPGQGLEWIGKIDPANGNTKYDQKFQGRATITADTSTNTAYLELSSLRSEDTAVYY
 
CARGYGSGFAYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTK
VDKKAEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSVMHEALHNH
YTQKSLSLSPGK
SEQ ID NO: 17 B8/B10/B12 light chain 
DIQMTQSPSSLSASVGDRVTITCRASQDISNHLNWYQQKPGKAPKLLIYYTSRIHSG
VPSSFRGSGSGTDYTLTISSLQPEDIATYFCQQGYTLPFTFGSGTKLEIKRTVAAPSVF
IFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
SEQ ID NO: 18 B9 heavy chain 
QVQLVQSGAEVKKPGASVKVSCKASGFNIKDIYIYWVRQAPGQGLEWIGKIDPANG
NTKYDQKFQGRATITADTSTNTAYLELSSLRSEDTAVYYCARGYGSGFAYWGQGTL
VTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKAEPKSCDKTHTCPP
CPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK
SEQ ID NO: 19 B9/B11 light chain 
QVQLVESGGGLVQPGGSLRLSCAASGSIFNIPVMGWYRQAPGKQRELVAGISTGGT
TNYGDSVKGRFTISRDNAKNTVYLQMNSLKPEDTAVYYCNVLVVSGIGSTLEVWGQ
GTLVTVSSGGGGSGGGGSDIQMTQSPSSLSASVGDRVTITCRASQDISNHLNWYQQ
KPGKAPKLLIYYTSRIHSGVPSSFRGSGSGTDYTLTISSLQPEDIATYFCQQGYTLPFT
FGSGTKLEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNR
GEC
SEQ ID NO: 20 B10 heavy chain 
QVQLVQSGAEVKKPGASVKVSCKASGFNIKDIYIYWVRQAPGQGLEWIGKIDPANG
NTKYDQKFQGRATITADTSTNTAYLELSSLRSEDTAVYYCARGYGSGFAYWGQGTL
VTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKAEPKSCDKTHTCP
PCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGKGGGG
SGGGGSQVQLVESGGGLVQPGGSLRLSCAASGSIFNIPVMGWYRQAPGKQRELVAG
ISTGGTTNYGDSVKGRFTISRDNAKNTVYLQMNSLKPEDTAVYYCNVLVVSGIGSTL
EVWGQGTLVTVSS
SEQ ID NO: 21 B12 heavy chain 
QVQLVESGGGLVQPGGSLRLSCAASGSIFNIPVMGWYRQAPGKQRELVAGISTGGT
TNYGDSVKGRFTISRDNAKNTVYLQMNSLKPEDTAVYYCNVLVVSGIGSTLEVWGQ
 
GTLVTVSSGGGGSQVQLVQSGAEVKKPGASVKVSCKASGFNIKDIYIYWVRQAPGQ
GLEWIGKIDPANGNTKYDQKFQGRATITADTSTNTAYLELSSLRSEDTAVYYCARG
YGSGFAYWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDK
KAEPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYT
QKSLSLSPGK
SEQ ID NO: 22 Linker1
   GGGGSGGGGS
SEQ ID NO: 23 Linker 2
   GGGGS
SEQ ID NO: 24 F4AM4-IgG1 heavy chain 
QVQLVQSGAEVKKPGASVKMSCKASGYTFTSSVMHWVRQAPGQGLEWIGYINPYT
DGTKYAQKFQGRATLTSDKSTSTAYMEFSSLRSEDTAVYYCGRPYYGTRYGSWFAY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKAEPKSC
DKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
SEQ ID NO: 25 F4AM4-IgG1 light chain

DIQMTQSPSSLSASVGDRVTITCRASQDISNYLNWYQQKPGKAPKLLIYYTSRLHSG
VPSRFSGSGSGTDYTLTISNLQPEDIATYYCQQGKNYPFTFGSGTKLEIKRTVAAPSV
FIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

## Claims

1. A bispecific antibody comprising a first antigen binding portion that binds to CD47 and a second antigen binding portion that binds to CLDN18.2, wherein the first antigen binding portion comprises a heavy chain variable region (VH) and a light chain variable region (VL),
the heavy chain variable region comprises:
1) HCDR1 comprising the amino acid sequence of SEQ ID NO: 4;
2) HCDR2 comprising the amino acid sequence of SEQ ID NO: 5; and
3) HCDR3 comprising the amino acid sequence of SEQ ID NO: 6; and
the light chain variable region comprises:
1) LCDR1 comprising the amino acid sequence of SEQ ID NO: 7;
2) LCDR2 comprising the amino acid sequence of SEQ ID NO: 8; and
3) LCDR3 comprising the amino acid sequence of SEQ ID NO: 9.

2. The bispecific antibody of claim 1, wherein
the heavy chain variable region comprises: 1) the amino acid sequence of SEQ ID NO: 10; or 2) an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO: 10; and/or
the light chain variable region comprises: 1) the amino acid sequence of SEQ ID NO: 11; or 2) an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO: 11;
preferably, the heavy chain variable region comprises the amino acid sequence of SEQ ID NO: 10, and the light chain variable region comprises the amino acid sequence of SEQ ID NO: 11.

3. The bispecific antibody of claim 1 or 2, wherein the second antigen binding portion comprises an immunoglobulin single variable domain (VHH) that binds to CLDN18.2;
preferably, the immunoglobulin single variable domain comprises:
CDR1 comprising the amino acid sequence of SEQ ID NO: 13;
CDR2 comprising the amino acid sequence of SEQ ID NO: 14; and
CDR3 comprising the amino acid sequence of SEQ ID NO: 15;
more preferably, the immunoglobulin single variable domain comprises: 1) the amino acid sequence of SEQ ID NO: 12; or 2) an amino acid sequence having at least 80%, at least 85%, at least 90%, at least 91%, at least 92%, at least 93%, at least 94%, at least 95%, at least 96%, at least 97%, at least 98%, or at least 99% sequence identity to the amino acid sequence of SEQ ID NO: 12.

4. The bispecific antibody of any one of claims 1-3, wherein the first antigen binding portion and the second antigen binding portion are connected through a linker.

5. The bispecific antibody of any one of claims 1-4, which further comprises an immunoglobulin heavy chain constant region (CH) and an immunoglobulin light chain constant region (CL);
preferably, the heavy chain constant region is a human IgG1 heavy chain constant region, and/or the light chain constant region is a human kappa light chain constant region;
more preferably, the heavy chain constant region comprises the amino acid sequence of SEQ ID NO: 2; and/or the light chain constant region comprises the amino acid sequence of SEQ ID NO: 3.

6. The bispecific antibody of claim 5, which comprises a first polypeptide and a second polypeptide, wherein
the first polypeptide has the following structure from the N-terminus to the C-terminus:
VH-CH-Linker-VHH,
the second polypeptide has the following structure from the N-terminus to the C-terminus:
VL-CL,
wherein
the VH and the VL are respectively the heavy chain variable region and the light chain variable region as defined in claim 1 or 2;
the VHH is the immunoglobulin single variable domain as defined in claim 3;
the CH and the CL are respectively the heavy chain constant region and the light chain constant region as defined in claim 5;
the Linker is a linker;
preferably, the first polypeptide comprises the amino acid sequence of SEQ ID NO: 20, and the second polypeptide comprises the amino acid sequence of SEQ ID NO: 17.

7. An isolated polynucleotide encoding the bispecific antibody of any one of claims 1-6.

8. An expression vector comprising the polynucleotide of claim 7.

9. A host cell comprising the polynucleotide of claim 7 or the expression vector of claim 8.

10. An antibody conjugate comprising the bispecific antibody of any one of claims 1-6 conjugated with at least one therapeutic agent.

11. A pharmaceutical composition comprising the bispecific antibody of any one of claims 1-6 or the antibody conjugate of claim 10, and a pharmaceutically acceptable carrier.

12. Use of the bispecific antibody of any one of claims 1-6, the antibody conjugate of claim 10 or the pharmaceutical composition of claim 11 in the preparation of a drug for treating cancer, preferably gastric cancer.
